# EUROPÄISCHE PATENTSCHRIFT

(11) **EP 1 341 457 B1**
(45) Veröffentlichungstag und Bekanntmachung des Hinweises auf die Patenterteilung: **22.09.2004**
(21) Anmeldenummer: 02764749.4
(22) Anmeldetag: 22.07.2002
(51) Int. Cl.: A61B 17/34

(54) **TROKARKOPF MIT KLAPPENVENTIL**
TROCAR HEAD COMPRISING A FLAP VALVE
TETE DE TROCART POURVUE D'UNE VALVE A CLAPET

(30) Priorität: 24.07.2001 DE 10135979
(43) Veröffentlichungstag der Anmeldung: 10.09.2003
(73) Patentinhaber: Karl Storz GmbH & Co. KG, 78532 Tuttlingen (DE)
(72) Erfinder: BACHER, Uwe, 78532 Tuttlingen (DE); DITTRICH, Horst, 78194 Immendingen (DE); OBERLÄNDER, Martin, 78532 Tuttlingen (DE)
(74) Vertreter: Hofmeister, Frank Horst
(86) Internationale Anmeldenummer: PCT/EP2002/008143
(87) Internationale Veröffentlichungsnummer: WO 2003/011153

(56) Entgegenhaltungen:
- DE-U- 8 916 160
- DE-U- 9 418 005
- DE-U- 29 700 762
- US-A- 6 066 117

## Beschreibung

Die Erfindung betrifft ein medizinisches Instrument mit einem in einem Gehäuse ausgebildeten hohlen Instrumentenkanal, der durch mindestens einen Ventilkörper verschließbar ist und durch ein in den Instrumentenkanal eingeschobenes Instrument in eine Offenstellung verschwenkbar ist, wobei der mindestens eine Ventilkörper als aus einem im wesentlichen biegesteifen Material bestehende Klappe ausgebildet ist, die über ein als Drahtfeder ausgebildetes biegeelastisches Verbindungselement derart verschwenkbar am Gehäuse gelagert ist, dass der Ventilkörper in Schließrichtung vorgespannt ist.

Derartige medizinische Instrumente werden beispielsweise als Trokare verwendet. Trokare dienen dazu, Operationsinstrumente für endoskopische Operationen beispielsweise in die Bauchhöhle eines Patienten einzuführen. Dazu wird die Trokarhülse an die Bauchdecke angesetzt, ein Trokardorn in den hohlen Instrumentenkanal eingeschoben, mit Hilfe des Trokardorns eine Öffnung in die Bauchdecke gestoßen und anschließend die Trokarhülse durch die Öffnung in den Bauchraum eingeführt. Der Trokardorn kann dann wieder aus der Trokarhülse herausgezogen werden. Da es bei endoskopischen Operationen des Bauchraums üblich ist, den Bauchraum zur Erweiterung des Operationsraums und Ausbildung eines Pneumoperitoneums mit Gas zu befüllen, ist der hohle Instrumentenkanal der Trokarhülse über einen Ventilkörper verschließbar, damit das Gas bei entnommenem Instrument nicht über die Trokarhülse aus dem Bauchraum entweichen kann. Die bekannten Ventilkörper sind dabei so ausgebildet, dass sie durch ein in den instrumentenkanal eingeschobenes Instrument geöffnet werden und sich beim Herausziehen des Instruments auch selbsttätig wieder schließen.

Ein gattungsgemäßes, als Trokarhülse ausgebildetes medizinisches Instrument ist beispielsweise aus der DE 297 00 762 U1 bekannt. Bei diesem bekannten Trokar ist das den Ventilkörper vorspannende Verbindungselement als U-förmig gebogene Drahtfeder ausgebildet. Die freien Enden der Feder sind an unterschiedlichen Stellen rechtwinklig nach innen umgebogen und in versetzt zueinander angeordneten Bohrungen im Gehäuse festgelegt. Aufgrund der unterschiedlichen Schenkellängen der Feder bewirkt das Verschwenken des Ventilkörpers ein Spannen der Feder, so dass diese den Ventilkörper in Richtung auf die Schließstellung belastet. Die versetzte Anordnung der Bohrungen zur Aufnahme der umgebogenen freien Ende der Feder macht es erforderlich, dass die Abwinklungen der freien Enden der Federschenkel an genau definierten Stellen ausgeführt werden müssen, da ansonsten die Feder nicht am Gehäuse festlegbar ist. Durch dieses exakte Einhalten enger Toleranzwerte ist die Herstellung der Feder arbeitsaufwendig und somit teuer. Darüber hinaus ist die Feder aufgrund der unterschiedlichen Schenkellängen nur in einer Position am Gehäuse festlegbar, wodurch die Montage dieses bekannten Trokars erschwert wird.

Ein weiteres, als Trokarhülse ausgebildetes medizinisches Instrument mit einem in dem Instrumentenkanal angeordneten Ventilkörper ist aus der DE-C1-43 06 205 bekannt. Bei diesem bekannten medizinischen Instrument ist der Ventilkörper als aus Silikon hergestellte Klappe ausgebildet, die über ein Filmscharnier verschwenkbar mit einem Tragkörper verbunden ist, der wiederum am Gehäuse so festlegbar ist, dass die Ventilklappe den Instrumentenkanal verschließen und beim Einschieben eines Instruments wieder freigeben kann. Zwar ist dieser bekannte Ventilkörper kostengünstig als Spritzgußteil herstellbar und darüber hinaus auch zu Reinigungs- und Austauschzwecken leicht auswechselbar, jedoch weist diese bekannte Konstruktion die Nachteile auf, dass einerseits die labile Silikonklappe nicht ausreichend abdichtet, da das Filmscharnier nur eine geringe Vorspannung der Ventilklappe in Schließrichtung bewirkt, und andererseits die Gefahr besteht, dass das Filmscharnier insbesondere bei exzentrischer Betätigung der angeschlossenen Ventilklappe einreißt.

Ferner ist ein als Trokar ausgebildetes medizinisches Instrument mit einem Ventilkörper zum Verschließen eines hohlen Instrumentenkanals ist aus der DE-C2-39 23 243 bekannt. Bei dieser bekannten Konstruktion besteht die Ventilklappe aus einem biegesteifen Material und kann über einen Stößel manuell geöffnet werden, damit beim Einführen eines spitzen Instruments in den Instrumentenkanal die Spitze des Instruments beim Aufstoßen der Ventilklappe nicht beschädigt wird und damit bei der Entnahme von Gewebestücken durch die Ventilklappe die Gewebeprobe nicht beschädigt wird bzw. nicht von der Faßzange abgestreift wird. Diese bekannte Konstruktion ist aber konstruktiv so aufwendig, dass die Ventilklappe nicht vom Anwender zu Reinigungs- oder Austauschzwecken entnommen werden kann.

Ausgehend von diesem Stand der Technik liegt der Erfindung die **Aufgabe** zugrunde, ein medizinisches Instrument der eingangs genannten Art so auszugestalten, dass es einfach und kostengünstig aufgebaut ist und der zuverlässig abdichtende Ventilkörper insbesondere zu Reinigungszwecken oder zum Auswechseln leicht demontierbar ist.

Die **Lösung** dieser Aufgabenstellung ist erfindungsgemäß dadurch gekennzeichnet, dass das Verbindungselement einerseits am Gehäuse festgelegt ist und andererseits den Ventilkörper trägt, wobei das Verbindungselement in einer im Gehäuse ausgebildeten Ringnut festgelegt ist.

Die Lagerung einerseits des Verbindungselements am Gehäuse und andererseits des Ventilkörpers am Verbindungselement erfolgt bei einer praktischen Ausführungsform der Erfindung über Ringnuten am Gehäuse und am Ventilkörper, wobei das vorzugsweise als mehrfach gebogene Drahtfeder ausgebildete Verbindungselement in der Ringnut am Gehäuse festlegbar ist und die Ringnut am Ventilkörper zur Aufnahme des Verbindungselements dient.

Der als Klappe ausgebildete Ventilkörper ist nur über das biegeelastische Verbindungselement mit dem Gehäuse verbunden, so dass es nur einer Demontage des Verbindungselements mitsamt dem Ventilkörper oder aber der Demontage des Ventilkörpers vom Verbindungselement bedarf, um den Ventilkörper zu Reinigungs- oder Austauschzwecken entnehmen zu können.

Bei einer bevorzugten Ausgestaltung der Erfindung ist die Ringnut als unterbrochene Ringnut ausgebildet. Die Unterbrechungen der Ringnut sind als Öffnungen der Ringnut in radialer Richtung so ausgebildet, dass im äußersten Fall die Ringnut aus nur wenigen Kreisringsegmenten besteht.

Um sicherzustellen, dass der Ventilkörper richtig positioniert am Gehäuse festgelegt wird, wenn die Ringnut zur Aufnahme des Verbindungselements nur aus einzelnen unterbrochenen Kreisringsegmenten besteht, ist am Verbindungselement mindestens ein mit mindestens einem Ringnutabschnitt zusammenwirkendes Positionierungselement angeordnet.

Die Verwendung von umlaufenden Ringnuten zur gehäuseseitigen und ventilköperseitigen Aufnahme des Verbindungselements stellt eine besonders einfach herstellbare und leicht montierbare/demontierbare Art der Festlegung des Verbindungselements dar, wodurch die praktische Handhabbarkeit eines solchermaßen ausgebildeten medizinischen Instruments deutlich erleichtert und verbessert wird.

Die aus Verbindungselement und Ventilkörper bestehende Baueinheit ist kostengünstig herstellbar. Das Verbindungselement und der Ventilkörper werden durch Klemmung über Formschluß verbunden. Der Ventilkörper kann dabei aus mindestens zwei Teilen aufgebaut sein, die nach ihrer gegenseitigen Verbindung miteinander, beispielsweise durch Verklemmen, Verschrauben oder Verkleben, zumindest teilweise einen dafür vorgesehenen Abschnitt des Verbindungselements umschließen. Ebenso kann ein einstückig ausgebildeter Ventilkörper über Klemmung mit dem entsprechenden Abschnitt des Verbindungselements verbunden werden. Gemäß einer weiteren Ausführungsform kann auch realisiert werden, dass der entsprechende Abschnitt des Verbindungselements in den Ventilkörper eingegossen ist. Durch diese kostengünstigen Herstellungsverfahren wird der Einsatz der Baueinheit als Wegwerfbauteil möglich.

In einer weiteren Ausführungsform der Erfindung ist der in der Ringnut am Gehäuse festlegbare Teil des Verbindungselements von einem Rohr aus einem inelastischen Material umgeben, wobei der Außendurchmesser des Verbindungselements nur geringfügig kleiner ist als der Innendurchmesser des Rohres und das Verbindungselement und das Rohr kraftschlüssig miteinander verbunden sind. Um diese kraftschlüssige Verbindung herzustellen, wird erfindungsgemäß vorgeschlagen, dass die beiden Bauteile durch Verquetschen oder Verschweißen, insbesondere Laserschweißen, fest miteinander verbunden sind und. Durch die Wahl der Lage der Quetschstellen können die Vorspannung und die Rückstellkraft des Ventilkörpers exakt den jeweiligen Anforderungen angepaßt werden.

Die Verwendung des in dem Rohr angeordneten Verbindungselements soll verhindern, dass beim Verschwenken des Ventilkörpers das in der Ringnut gelagerte Verbindungselement umschlägt.

Das Verbindungselement wird bevorzugt aus elastischen Materialien wie Federstahl oder superelastischen Legierungen, wie beispielsweise Ni-Ti- oder Cu-Al-Ni- oder Cu-Zn-Al-Legierungen, hergestellt. Durch die Wahl des Materials des Verbindungselements wird eine Optimierung der Vorspannung und der Dichtigkeit ermöglicht. Diese Vorspannung bewirkt, dass sich der Ventilkörper nach dem Herausziehen des Instruments aus dem Instrumentenkanal automatisch und mit einem ausreichenden Anpressdruck wieder auf den Ventilsitz legt, um den Instrumentenkanal zuverlässig abzudichten.

Gemäß einer weiteren Ausführungsform der Erfindung wird vorgeschlagen, dass das Verbindungselement mehradrig aus mindestens zwei miteinander verdrehten Adern bestehend aufgebaut ist und die einzelnen Adern des Verbindungselements an einzelnen Stellen durch Verquetschen oder Verschweißen miteinander verbunden sind. Die Verwendung des aus mehreren miteinander verdrehten Adern bestehenden Drahtes verleiht dem Verbindungselement die notwendige Elastizität, um einerseits das Ausschwenken des Ventilkörpers zu ermöglichen und andererseits einen festen und stabilen Halt des Verbindungselements in der Ringnut zu gewährleisten.

Der erfindungsgemäße Ventilkörper besteht vorzugsweise aus einem Hartkunststoff.

Zur Verbesserung der Abdichtung des Instrumentenkanals über den Ventilkörper wird erfindungsgemäß vorgeschlagen, dass am Ventilkörper ein Dichtring, insbesondere aus einem Elastomer-Kunststoff, festlegbar ist und am Gehäuse eine den Instrumentenkanal koaxial umgebende Dichtfläche zur Auflage des Dichtringes des Ventilkörpers ausgebildet ist. Diese Dichtfläche ist gemäß praktischen Ausführungsformen der Erfindung vorzugsweise als erhöhte Dichtkante oder plane Fläche ausgebildet. Ebenso kann die im wesentlichen parallel zur Ventilebene ausgebildete Dichtfläche durch eine Stufe begrenzt sein, wobei der Verlauf der Stufe der äußeren Form des Dichtringes in axialer Richtung entspricht. Die vorzugsweise vorgesehene Auswechselbarkeit des am Ventilkörper gelagerten Dichtrings erleichtert einerseits die Reinigung und andererseits bei geringen Kosten das Beseitigen von Dichtproblemen durch bloßes Auswechseln des Dichtrings.

Um zu vermeiden, dass empfindliche Instrumentenspitzen dadurch beschädigt werden, dass sie beim Einführen des Instruments in den Instrumentenkanal den Ventilkörper aufstoßen müssen und damit bei der Entnahme von Gewebestücken die Gewebeprobe durch die Ventilklappe nicht beschädigt wird bzw. nicht von der Faßzange abgestreift wird, wird mit der Erfindung weiterhin vorgeschlagen, dass der Ventilköper zusätzlich über einen manuell betätigbaren Mechanismus in die Offenstellung verlagerbar ist. Mittels dieses Mechanismus besteht nunmehr die Möglichkeit, alternativ zum Aufstoßen des Ventilkörpers mit dem in den Instrumentenkanal eingeführten Instrument, den Ventilkörper von außen manuell zu öffnen, um so den Kontakt der Instrumentenspitze und/oder der Gewebeprobe mit dem Ventilkörper zu verhindern.

Gemäß einer praktischen Ausführungsform der Erfindung ist der manuell betätigbare Mechanismus als exzentrisch, insbesondere parallel, zum Instrumentenkanal angeordneter Stößel ausgebildet ist, der über ein am Gehäuse gelagertes Betätigungselement in Längsrichtung des Instrumentenkanals verschiebbar ist. Die Verwendung des erfindungsgemäßen Verbindungselements ist insbesondere bei dieser Ausgestaltung vorteilhaft, da dieses Verbindungselement eine ausreichende Stabilität gegenüber der exzentrischen Belastung durch den Stößel aufweist.

Mit einer ersten Ausführungsform der Erfindung wird vorgeschlagen, dass das Betätigungselement als parallel zum Instrumentenkanal verschiebbare und über eine Feder in Schließrichtung des Ventilkörpers vorgespannte Scheibe ausgebildet ist. Die Vorspannung des Betätigungselements in Schließrichtung des Ventilkörpers gewährleistet, dass das Betätigungselement in einer Ruheposition gehalten wird, in der der Ventilkörper geschlossen ist.

Bei einer zweiten erfindungsgemäßen Ausführungsform wird das Betätigungselement zum Verschieben des Stößels von einem verschwenkbar am Gehäuse gelagerten Hebel gebildet, der vorteilhafterweise über eine Feder in Schließrichtung des Ventilkörpers vorgespannt ist.

Die Ebene der Ventilöffnung zur Längsachse des Instrumentenkanals kann vorteilhafterweise in weiten Bereichen variiert werden und beträgt vorzugsweise 90°.

Schließlich wird mit der Erfindung vorgeschlagen, dass am Gehäuse ein weiteres Bauteil derart festlegbar ist, dass das weitere Bauteil die Ringnut bzw. die Ringnutabschnitte zumindest teilweise verschließend das Verbindungselement in der Ringnut bzw. den Ringnutabschnitten hält, wobei das Gehäuse vorteilhafterweise ein Trokarkopf und das weitere Bauteil eine am Trokarkopf festlegbare Trokarhülse eines Trokars ist.

Weitere Merkmale und Vorteile der Erfindung ergeben sich anhand der nachfolgenden Beschreibung der zugehörigen Zeichnung, in der vier Ausführungsbeispiele eines erfindungsgemäßen medizinischen Instruments nur beispielhaft schematisch dargestellt sind. In der Zeichnung zeigt:
- Fig. 1: einen Längschnitt durch eine erste Ausführungsform eines erfindungsgemäßen, als Trokar ausgebildeten medizinischen Instruments;
- Fig. 2a: eine perspektivische Ansicht des Trokarkopfes des Trokars gemäß Fig. 1 mit in den Instrumentenkanal eingesetztem Trokardorn und mit in die Offenstellung verschwenktem Ventilkörper;
- Fig. 2b: eine Fig. 2a entsprechende perspektivische Ansicht, jedoch ohne Trokardorn, sowie den Ventilkörper in der geschlossenen Stellung darstellend;
- Fig. 3: einen schematischen Längsschnitt entlang der Schnittlinie III-III gemäß Fig. 2a;
- Fig. 4: einen schematischen Längsschnitt entlang der Schnittlinie IV-IV gemäß Fig. 2b;
- Fig. 5: eine schematische perspektivische Ansicht des Verbindungselements mit daran festgelegtem Ventilkörper gemäß einer ersten erfindungsgemäßen Ausführungsform;
- Fig. 6: eine Fig.5 entsprechende perspektivische Ansicht, jedoch eine zweite erfindungsgemäße Ausführungsform des Verbindungselements darstellend;
- Fig. 7: einen Fig. 3 entsprechenden schematischen Längsschnitt, jedoch eine zweite erfindungsgemäße Ausführungsform darstellend;
- Fig. 8: einen Fig. 7 entsprechenden schematischen Längsschnitt, jedoch eine dritte erfindungsgemäße Ausführungsform darstellend;
- Fig. 9a: eine schematische perspektivische Ansicht eines Trokarkopfes mit geschlossenem Ventilkörper, eine vierte erfindungsgemäße Ausführungsform des Verbindungselements darstellend;
- Fig. 9b: einen schematischen Längsschnitt entlang der Schnittlinie IXb-IXb gemäß Fig. 9a;
- Fig. 10a: eine Fig. 9a entsprechende perspektivische Ansicht, jedoch den Ventilkörper in der offenen Stellung darstellend;
- Fig. 10b: einen schematischen Längsschnitt entlang der Schnittlinie Xb-Xb gemäß Fig. 10a
- Fig. 11: eine Explosionsdarstellung des Ventilkörpers gemäß Fig. 9a bis 10b.

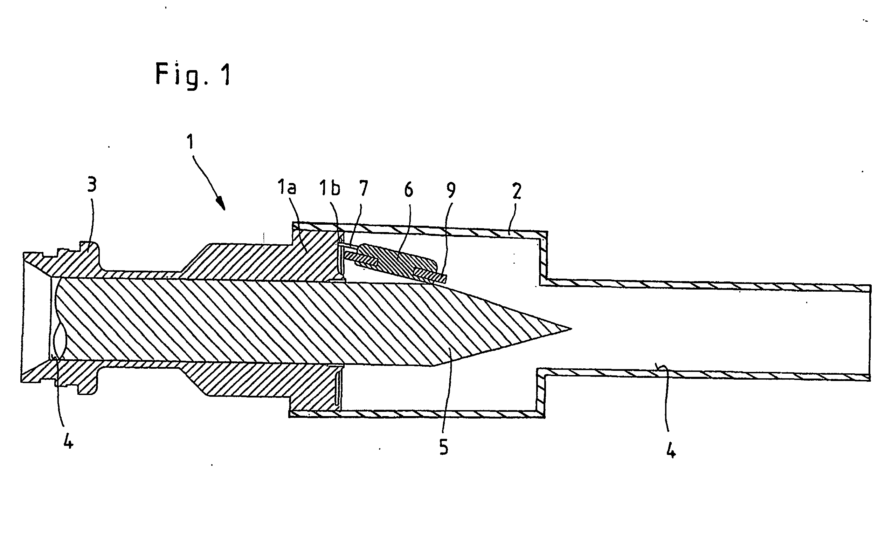

Die Abbildung Fig. 1 zeigt im Längsschnitt einen aus einem Trokarkopf 1 und einer Trokarhülse 2 bestehenden Trokar. Der Trokarkopf 1 weist am proximalen Ende eine Handhabe 3 auf und wird in Längsrichtung von einem zentral angeordneten hohlen Instrumentenkanal 4 durchzogen, der sich in der Trokarhülse 2 fortsetzt.

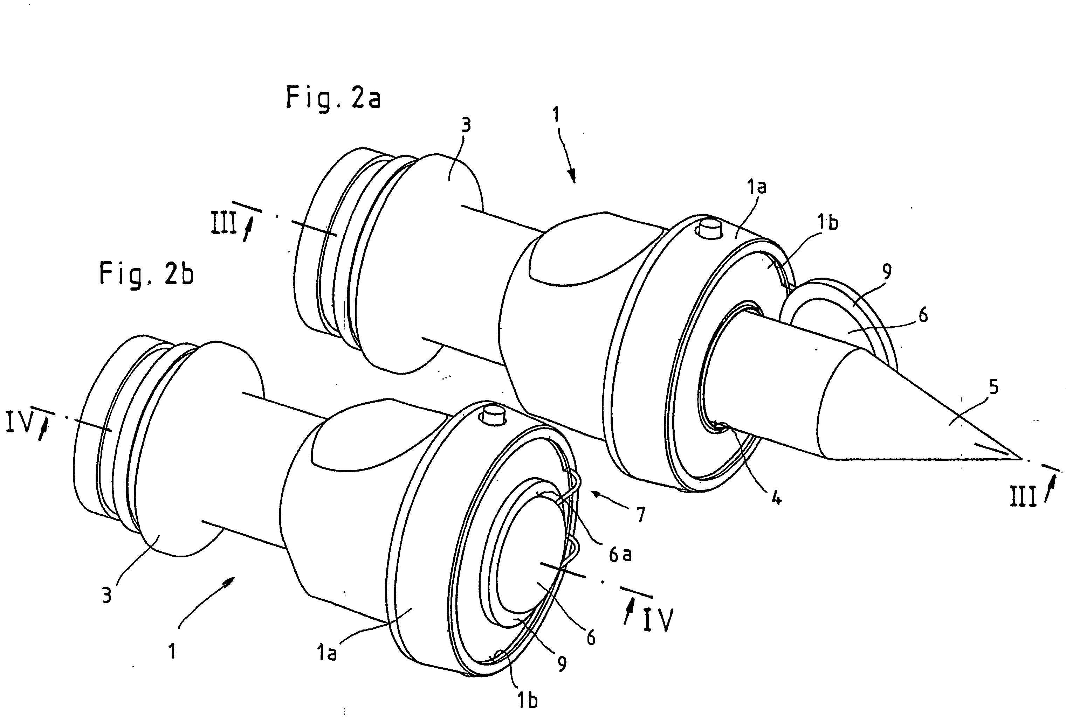

Die Abbildungen Fig. 2a und Fig. 2b zeigen in perspektivischer Ansicht in zwei unterschiedlichen Betriebsstellungen den Trokarkopf 1 des Trokars zum Einführen endoskopischer Operationsinstrumente gemäß Fig.1.

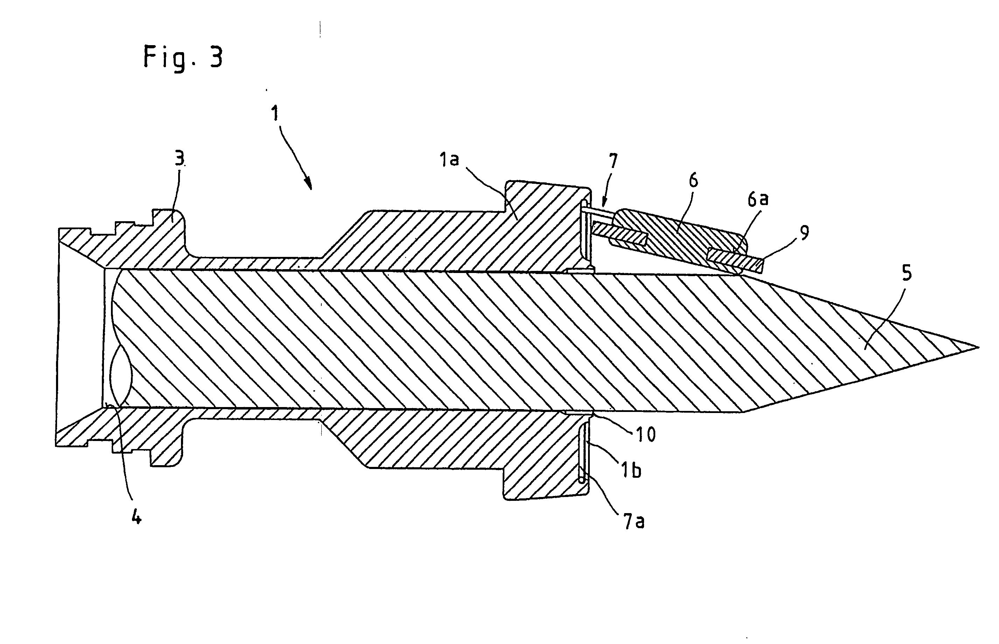

Der hohle Instrumentenkanal 4 dient dazu, um zu Beginn der Operation einen Trokardorn 5 aufzunehmen, wie dieser in Fig. 1, 2a und 3 dargestellt ist, mit dem eine Öffnung, beispielsweise in die Bauchdecke des Patienten gestoßen wird. In diese Öffnung wird dann die Trokarhülse 2 eingeschoben und der Trokardom 5 anschließend wieder aus dem Instrumentenkanal 4 herausgezogen. Während der nachfolgenden Operation können dann über der Instrumentenkanal 4 die unterschiedlichsten endoskopischen Instrumente in das Operationsgebiet eingeführt werden. Da es bei endoskopischen Operationen des Bauchraums üblich ist, den Bauchraum des Patienten zur Erweiterung des Operationsraums und Ausbildung eines Pneumoperitoneums mit Gas zu befüllen, ist der hohle Instrumentenkanal 4 des Trokars über einen Ventilkörper 6 verschließbar, um das Austreten des Gases beim Herausziehen eines Instruments aus dem Instrumentenkanal 4 zu verhindern.

Der als Ventilklappe ausgebildete Ventilkörper 6 ist bei den dargestellten Ausführungsbeispielen über ein als Federdraht ausgebildetes biegeelastisches Verbindungselement 7 am Gehäuse 1a des Trokarkopfes 1 verschwenkbar gelagert. Der solchermaßen am Gehäuse 1a gelagerte Ventilkörper 6 ist zwischen einer Offenstellung (Fig. 1, 2a, 3, 7, 8 und 10a) und einer geschlossenen Stellung (Fig. 2b, 4 und 9a) verschwenkbar.

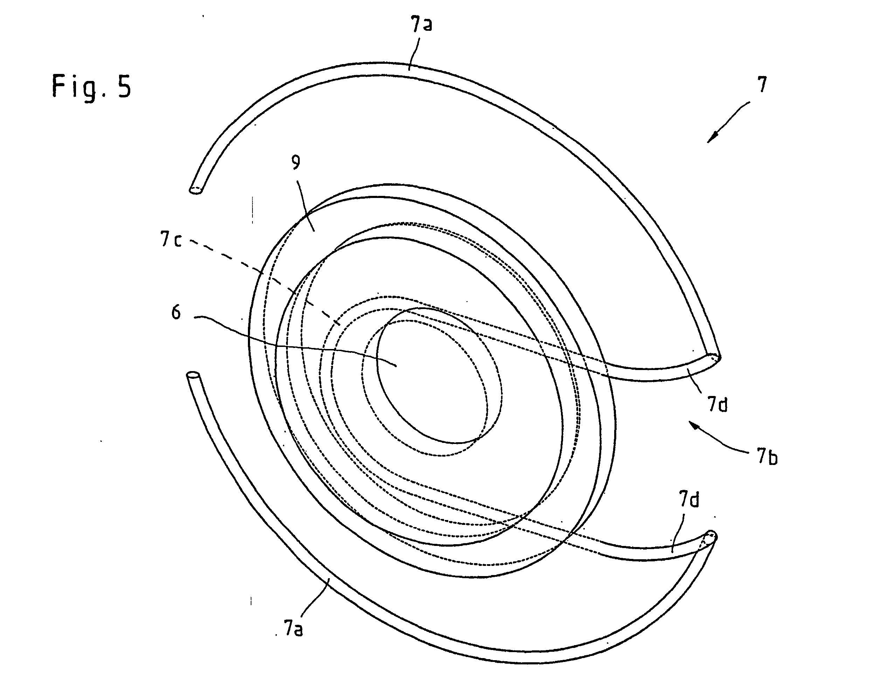

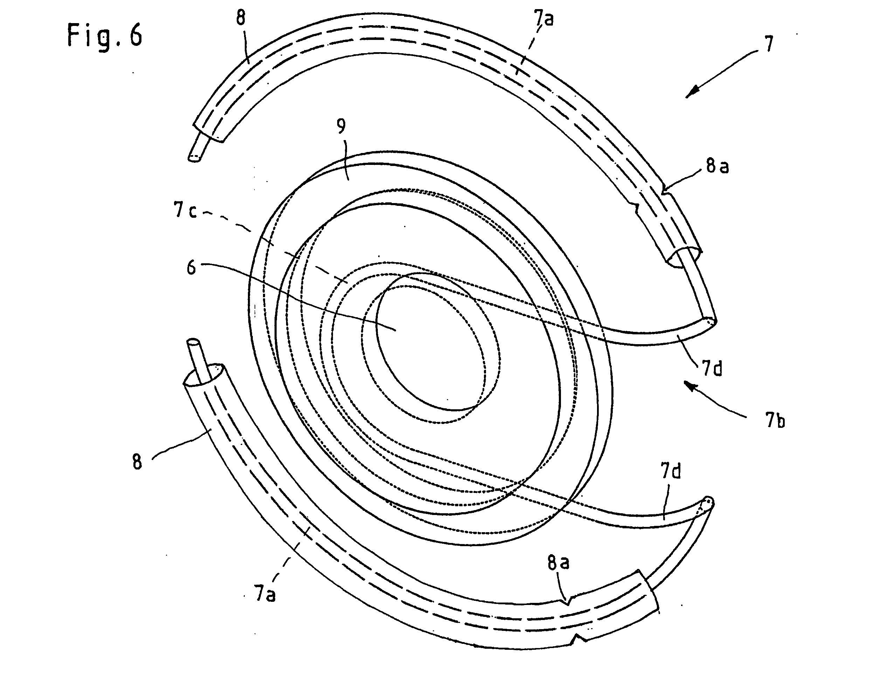

Zum Festlegen des Verbindungselements 7 am Gehäuse 1 a des Trokarkopfes 1 dient die im Gehäuse 1a ausgebildete Ringnut 1b. Wie insbesondere aus den Schnittdarstellungen gemäß Fig. 1, 3, 4, 7 und 8 ersichtlich, ist das als Federdraht ausgebildete Verbindungselement 7 in der eine Hinterschneidung bildenden Ringnut 1b des Gehäuses 1a festgelegt. Zum Festlegen des Ventilkörpers 6 am Verbindungselement 7 dient bei den Ausführungsbeispielen gemäß den Abbildungen Fig. 1 bis 8 eine im Ventilkörper 6 ausgebildete Ringnut 6a. Die Aufnahme des Verbindungselements 7 in der Ringnut 6a des Ventilkörpers 6 ergibt sich am deutlichsten aus den schematischen Darstellungen gemäß Fig. 5 und 6.

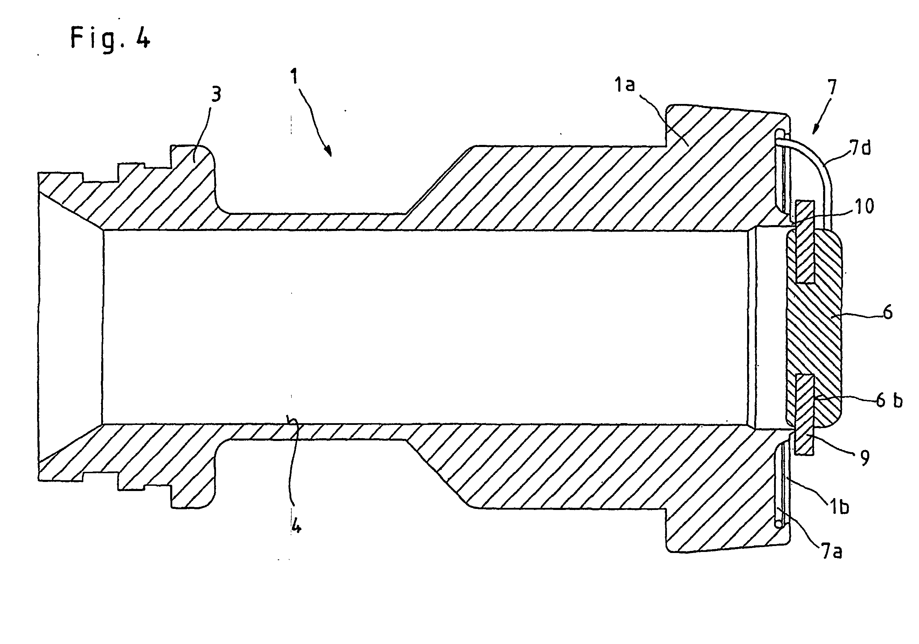

Die Form der Verbindungselemente 7 der dargestellten Ausführungsformen gemäß den Abbildungen Fig. 1 bis 8 ergibt sich am besten aus einer Zusammenschau der Abbildungen Fig. 2b, 4, 5 und 6. Der in der Ringnut 1b des Gehäuses 1a festgelegte Teil des das Verbindungselement 7 bildenden Federdrahts besteht bei der in Fig. 5 dargestellten ersten Ausführungsform aus zwei halbkreisförmig ausgebildeten Kreisabschnitten 7a, die über einen im wesentlichen rechtwinklig nach oben aus der Ebene der Kreisabschnitte 7a herausgebogenen Abschnitt 7b miteinander verbunden sind. Um eine Schlaufe 7c zur Aufnahme in der Ringnut 6a des Ventilkörpers 6 zu bilden, weist der Abschnitt 7b eine bogenfömige Abwinklung 7d um ca. 90° auf, so dass die Schlaufe 7c, wie aus Fig. 4 ersichtlich, in der geschlossenen Stellung des Ventilkörpers 6 parallel zur Ebene der Kreisabschnitte 7a angeordnet ist. Aufgrund der Materialsteifigkeit des mehrfach gebogenen Federdrahts ist der in der Schlaufe 7c festgelegte Ventilkörper 6 in Schließrichtung vorgespannt. Diese Vorspannung des Ventilkörpers 6 sorgt dafür, dass sich der Ventilkörper 6 nach dem Herausziehen des Instruments aus dem Instrumentenkanal 4 automatisch wieder abdichtend vor die Öffnung des Instrumentenkanals 4 legt. Diese Vorspannung kann durch eine Verkleinerung des Winkels zwischen dem Abschnitt 7b und den bogenförmigen Abwinklungen erhöht werden.

Die in Fig. 6 dargestellte zweite Ausführungsform des Verbindungselements 7 unterscheidet sich von der Ausführungsform gemäß Fig. 5 dadurch, dass die in der Ringnut 1b des Gehäuses 1a festlegbaren Kreisabschnitte 7a des Verbindungselements 7 von Röhren 8 aus einem inelastischen Material umgeben sind, wobei die Röhren 8 kraftschlüssig mit den Kreisabschnitten 7a des Verbindungselements 7 verbunden sind. Durch die Lagerung des Verbindungselements 7 in dem Rohr 8 und die punktuelle kraftschlüssige Verbindung der beiden Bauteile wird auf einfache Weise erreicht, dass das Verbindungselement 7 einerseits ausreichend Flexibilität besitzt, um das Verschwenken des Ventilkörpers 6 zu ermöglichen, andererseits das Verbindungselement 7 aber nicht innerhalb der Ringnut 1b umschlagen kann, wenn der Draht beim Verschwenken des Ventilkörpers 6 tordiert wird.

Im dargestellten Fall wird die kraftschlüssige Verbindung durch Verquetschen erzielt. Durch die Wahl und Lage der Quetschstellen 8a kann die Vorspannung und die Rückstellkraft des Ventilkörpers 6 den jeweiligen Anforderungen angepaßt werden.

Alternativ zum Verquetschen von Rohr 8 und Verbindungselement 7 können das Rohr 8 und das Verbindungselement 7 auch miteinander verschweißt werden. Hierzu eignet sich insbesondere das Laserschweißen, da hierbei an vorgegebenen Stellen punktgenau eine Schweißung vorgenommen werden kann. Zu diesem Zweck wird an den auszubildenden Schweißpunkten ein bis zum Verbindungselement 7 reichendes Loch in das Rohr 8 gebohrt und das Rohr 8 in dieser Bohrung mit dem Verbindungselement 7 verschweißt.

Um für eine sichere und zuverlässig abdichtende Anlage des Ventilkörpers 6 an dem den Instrumentenkanal 4 umgebenden Gehäuse 1a zu sorgen, ist einerseits am Ventilkörper 6 auswechselbar ein Dichtring 9 in einer zweiten Ringnut 6b angeordnet und weist andererseits das Gehäuse 1a eine den Instrumentenkanal 4 koaxial umgebende, als erhöhte Dichtkante 10 ausgebildete Dichtfläche auf, auf der der Ventilkörper 6 mit dem Dichtring 9 aufliegt, wie dies die Abbildung Fig. 4 zeigt.

Bei den in den Abbildungen Fig. 1 bis 4 dargestellten ersten Ausführungsformen erfolgt das Öffnen des Ventilkörpers 6 ausschließlich dadurch, dass durch den Instrumentenkanal 4 ein Instrument hindurch geschoben wird und dieses Instrument mit seiner Spitze den Ventilkörper 6 aufstößt und in die Offenstellung verschwenkt, wie dies die Abbildungen Fig. 1, 2a und 3 zeigen.

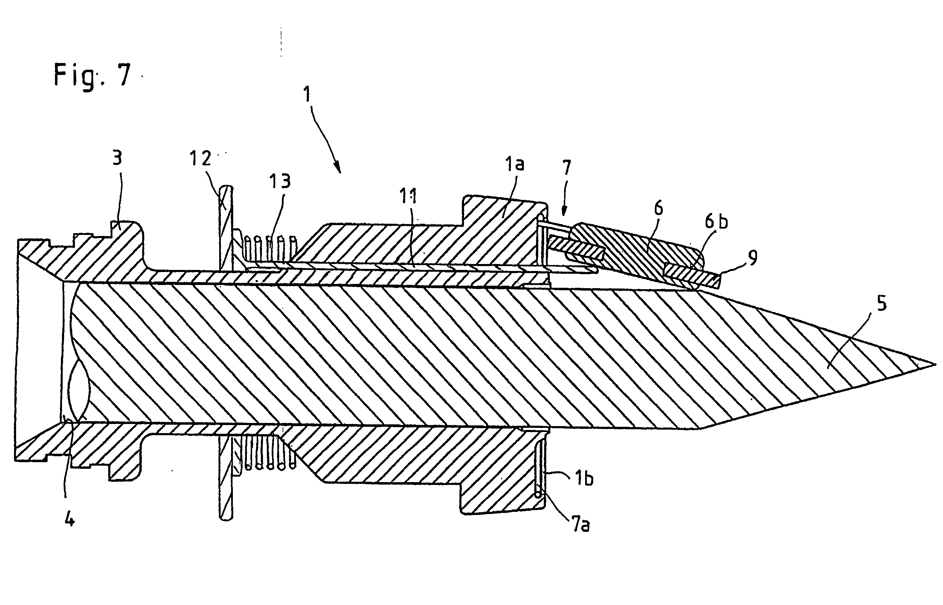

Da bei Instrumenten mit besonders scharfen und/oder empfindlichen Spitzen die Gefahr besteht, dass diese Spitzen beim Aufstoßen des Ventilkörpers 6 stumpf werden oder sogar beschädigt werden und damit bei der Entnahme von Gewebestücken durch die Ventilklappe die Gewebeprobe nicht beschädigt wird bzw. nicht von der Faßzange abgestreift wird, besteht bei den in Fig. 7 bis 11 dargestellten Ausführungsformen die Möglichkeit, den Ventilkörper 6 über einen manuell betätigbaren Mechanismus zu öffnen. Bei der in Fig. 7 dargestellten zweiten Ausführungsform eines Trokars besteht dieser Mechanismus aus einem exzentrisch und parallel zum Insrumentenkanal 4 im Gehäuse 1a gelagerten Stößel 11, der über ein als Scheibe 12 ausgebildetes, am Gehäuse 1a gelagertes Betätigungselement in Längsrichtung des Instrumentenkanals 4 verschiebbar ist. Um ein versehentliches Öffnen des Ventilkörpers 6 über den Stößel 11 zu vermeiden, ist die Scheibe 12 zur Betätigung des Stößels 11 über eine Feder 13 in Schließrichtung des Ventilkörpers 6 vorgespannt.

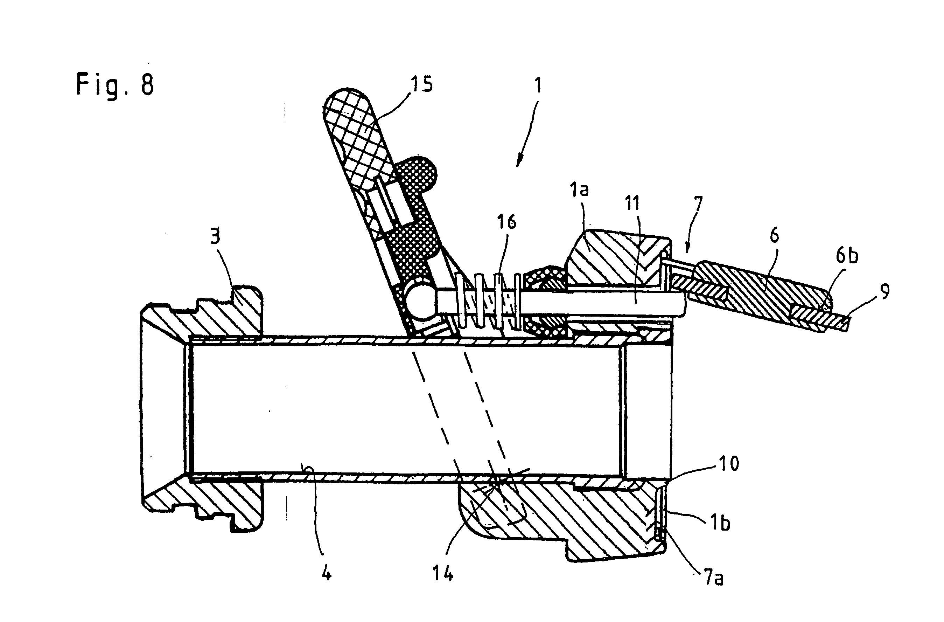

Bei der dritten Ausführungsform gemäß Fig. 8 ist das Betätigungselement zum Verschieben des Stößels 11 als um einen Drehpunkt 14 verschwenkbar am Gehäuse 1a gelagerter Hebel 15 ausgebildet. Auch bei dieser Ausgestaltungsform ist eine Feder 16 vorgesehen, um den Hebel 15 in Schließrichtung des Ventilkörpers 6 vorzuspannen.

Neben der dargestellten Möglichkeit, das Verbindungselement 7 über das Festlegen der Schlaufe 7c in der Ringnut 6a des Ventilkörpers 6 mit dem Ventilkörper 6 zu verbinden, besteht auch die Möglichkeit, dass das Verbindungselement 7 in das Material des Ventilkörpers 6 eingegossen wird, um so eine dauerhafte Verbindung herzustellen. Ebenso kann der Ventilkörper 6 aus mindestens zwei Teilen aufgebaut sein, die nach ihrer gegenseitigen Verbindung miteinander die Schlaufe 7c des Verbindungselements 7 umschließen.

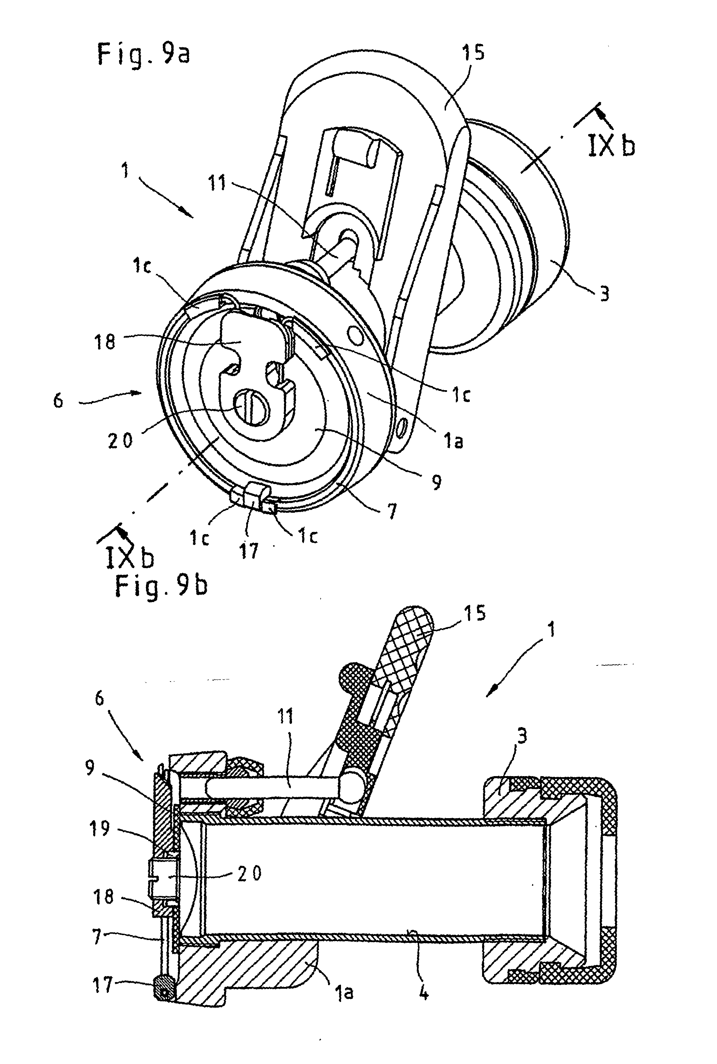

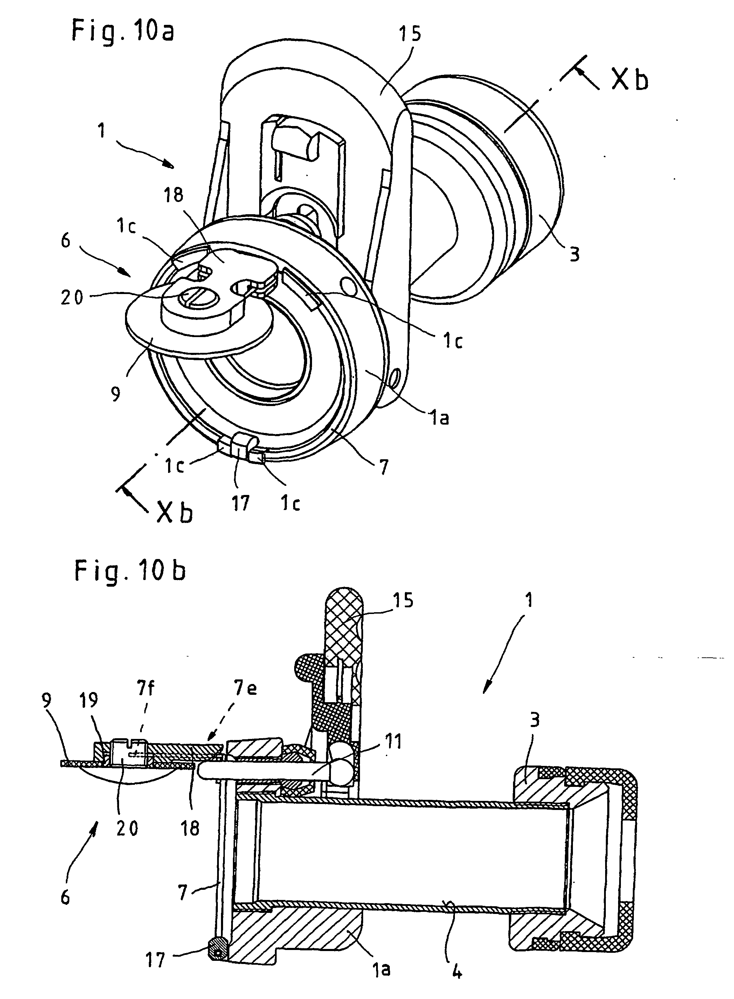

Die in den Abbildungen Fig. 9a bis 11 dargestellte vierte Ausführungsform unterscheidet sich von den zuvor beschriebenen Ausführungsformen im wesentlichen dadurch, dass die Ringnut 1b zur Aufnahme des Verbindungselements 7 nicht als zusammenhängende Nut, sondern unterbrochen so ausgebildet ist, dass die Ringnut 1b nur noch aus wenigen Kreisringsegmente darstellenden Ringnutabschnitten 1c besteht. Wie aus Fig. 9b und 10b ersichtlich, besteht die Ringnut 1b bei dieser Ausführungsform aus nur vier Ringnutabschnitten 1c.

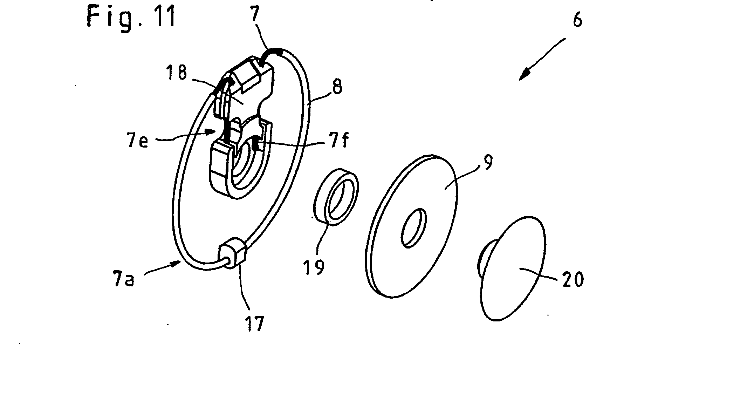

Wie insbesondere aus Fig. 11 ersichtlich, besteht der in den Ringnutabschnitten 1c des Gehäuses 1a festgelegte Teil des das Verbindungselement 7 bildenden Federdrahts aus einem fast vollständig geschlossenen Kreisabschnitt 7a und einem radial nach innen umgebogenen Abschnitt 7e, über den die Enden 7f des Federdrahts am Ventilkörper 6 festgelegt sind. Auch bei dieser Ausführungsform ist das Verbindungselement 7 teilweise in einem Rohr 8 gelagert.

Zum lagegerechten Positionieren des Verbindungselements 7 sowie des mit dem Verbindungselement 7 verbundenen Ventilkörpers 6 am Gehäuse 1a ist am Verbindungselement 7 mindestens ein Positionierungselement 17 angeordnet, das mit mindestens einem Ringnutabschnitt 1c der Ringnut 1b zusammenwirkt. Beim dargestellten Ausführungsbeispiel weist das Verbindungselement 7 ein Positionierungselement 17 auf, das so zwischen zwei Ringnutabschnitten 1c angeordnet ist, dass das Verbindungselement 7 in dieser Position nicht mehr relativ zum Gehäuse 1a verschiebbar ist.

Wie aus Fig. 1 ersichtlich, ist die Trokarhülse 2 derart am Gehäuse 1a des Trokarkopfes 1 festlegbar, dass die Trokarhülse 2 die Ringnut 1b bzw. die Ringnutabschnitte 1c zumindest teilweise so verschließt, dass das Verbindungselement 7 in der Ringnut 1b bzw. den Ringnutabschnitten 1c gehalten wird und nicht aus der Ringnut 1b bzw. den Ringnutabschnitten 1c herausspringen kann.

Der am Verbindungselement 7 festzulegende Ventilkörper 6 ist bei dieser vierten Ausführungsform, wie aus Fig. 11 ersichtlich, vierteilig aufgebaut und besteht aus einer auf die Enden 7f des Verbindungselements 7 aufsetzbaren Dichtungshalterung 18, einem Klemmsegment 19, dem Dichtring 9 sowie einer Schraube 20, über die das Klemmsegment 19 und der Dichtring 9 an der Dichtungshalterung 18 festlegbar sind. Beim Anziehen der Schraube 20 werden die Enden 7f des Verbindungselements 7 in der Dichtungshalterung 18 so verklemmt, dass der Ventilkörper 6 lagesicher am Verbindungselement 7 gelagert ist.

Der Aufbau und die Arbeitsweise des Ventilkörpers 6 ist insbesondere den Schnittdarstellungen gemäß Fig. 9b und 10b zu entnehmen. Diese Darstellungen zeigen zusammen mit den perspektivischen Darstellungen gemäß Fig. 9a und 10a auch, dass bei dieser Ausführungsform die Betätigung des Ventilkörper 6, ähnlich wie bei der Ausführungsform gemäß Fig. 8, über einen am Gehäuse 1a gelagerten Hebel 15 erfolgt, der einen Stößel 11 antreibt.

Obwohl bei den dargestellten Ausführungsbeispielen immer nur ein Ventilköper 6 vorgesehen ist, um den Instrumentenkanal 4 zu verschließen, ist es selbstverständlich auch möglich, in derselben Ebene des Instrumentenkanals 4 mehrere Ventilkörper 6 anzuordnen, die den Querschnitt des Instrumentenkanals 4 jeweils nur teilweise verschließen und die so angeordnet sind, dass sich die Ventilkörper 6 in der Schließstellung einander teilweise überlappen und gemeinsam den Querschnitt des Instrumentenkanals 4 verschließen.

Alternativ zu den dargestellten Ausführungsformen, bei denen das Verbindungselement 7 jeweils als einadriger Federdraht ausgebildet ist, ist es selbstverständlich auch möglich, das Verbindungselement 7 als aus mehreren miteinander verdrehten Adern hergestellten Federdraht auszubilden. Vorzugsweise sind bei dieser nicht dargestellten Ausführungsform die einzelnen Adern des Verbindungselements 7 an einzelnen Stellen durch Verquetschen oder Verschweißen miteinander verbunden, um ein zu weites Aufspleißen des Drahtes beim Verschwenken des Ventilkörpers 6 zu vermeiden.

Die dargestellte Konstruktion des Ventilkörpers 6 zeichnet sich dadurch aus, dass der Ventilkörper 6 über das Verbindungselement 7 in Schließrichtung vorgespannt ist und somit eine sichere Abdichtung des Instrumentenkanals 4 gewährleistet ist. Weiterhin ist der konstruktiv einfache Aufbau der Lagerung des Ventilkörpers 6 vorteilhaft, da dieser somit einfach und schnell zu Reinigungs- und Austauschzwecken demontiert und montiert werden kann.

### Bezugszeichenliste

- 1: Trokarkopf
- 1a: Gehäuse
- 1b: Ringnut
- 1c: Ringnutabschnitt
- 2: Trokarhülse/weiteres Bauteil
- 3: Handhabe
- 4: Instrumentenkanal
- 5: Trokardorn
- 6: Ventilkörper
- 6a: Ringnut
- 6b: Ringnut
- 7: Verbindungselement
- 7a: Kreisabschnitt
- 7b: Abschnitt
- 7c: Schlaufe
- 7d: Abwinklung
- 7e: Abschnitt
- 7f: Ende
- 8: Rohr
- 8a: Quetschstelle
- 9: Dichtring
- 10: Dichtkante
- 11: Stößel
- 12: Scheibe
- 13: Feder
- 14: Drehpunkt
- 15: Hebel
- 16: Feder
- 17: Positionierungselement
- 18: Dichtungshalterung
- 19: Klemmsegment
- 20: Schraube

## Patentansprüche

1. Medizinisches Instrument mit einem in einem Gehäuse (1a) ausgebildeten hohlen Instrumentenkanal (4), der durch mindestens einen Ventilkörper (6) verschließbar ist und durch ein in den Instrumentenkanal (4) einschiebbares Instrument in eine Offenstellung verschwenkbar ist, wobei der mindestens eine Ventilkörper (6) als aus einem im wesentlichen biegesteifen Material bestehende Klappe ausgebildet ist, die über ein als Drahtfeder ausgebildetes biegeelastisches Verbindungselement (7) derart verschwenkbar am Gehäuse (1a) gelagert ist, dass der Ventilkörper (6) in Schließrichtung vorgespannt ist,
**dadurch gekennzeichnet,**
**dass** das Verbindungselement (7) einerseits am Gehäuse (1a) festgelegt ist und andererseits den Ventilkörper (6) trägt, wobei das Verbindungselement (7) in einer im Gehäuse (1a) ausgebildeten Ringnut (1b) festgelegt ist.

2. Medizinisches Instrument nach Anspruch 1, **dadurch gekennzeichnet, dass** die im Gehäuse (1a) ausgebildete Ringnut (1b) zum Festlegen des Verbindungselements (7) als unterbrochene Ringnut ausgebildet ist.

3. Medizinisches Instrument nach Anspruch 2, **dadurch gekennzeichnet, dass** am Verbindungselement (7) mindestens ein mit mindestens einem Ringnutabschnitt (1c) zusammenwirkendes Positionierungselement (17) angeordnet ist.

4. Medizinisches Instrument nach Anspruch 1, **dadurch gekennzeichnet, dass** die im Gehäuse (1a) ausgebildete Ringnut (1b) zum Festlegen des Verbindungselements (7) als umlaufende Ringnut ausgebildet ist.

5. Medizinisches Instrument nach einem der Ansprüche 1 bis 4, **dadurch gekennzeichnet, dass** der Ventilkörper (6) eine Ringnut (6a), insbesondere eine umlaufende Ringnut, zur Aufnahme des Verbindungselements (7) aufweist.

6. Medizinisches Instrument nach einem der Ansprüche 1 bis 5, **dadurch gekennzeichnet, dass** der Ventilkörper (6) aus mindestens zwei Teilen besteht, die nach ihrer Verbindung miteinander, insbesondere durch Verklemmen, Verschrauben oder Verkleben, einen entsprechenden Abschnitt (7c, 7e) des Verbindungselements (7) umschließen.

7. Medizinisches Instrument nach einem der Ansprüche 1 bis 6, **dadurch gekennzeichnet, dass** das Verbindungselement (7) in das Material des Ventilkörpers (6) eingearbeitet, insbesondere eingegossen, ist.

8. Medizinisches Instrument nach einem der Ansprüche 1 bis 7 **dadurch gekennzeichnet, dass** der in der Ringnut (1b) im Gehäuse (1a) festlegbare Teil des Verbindungselements (7) von einem Rohr (8) aus einem inelastischen Material umgeben ist, wobei das Verbindungselement (7) und das Rohr (8) kraftschlüssig miteinander verbunden sind.

9. Medizinisches Instrument nach Anspruch 8, **dadurch gekennzeichnet, dass** das Verbindungselement (7) und das Rohr (8) miteinander verquetscht sind.

10. Medizinisches Instrument nach Anspruch 8, **dadurch gekennzeichnet, dass** das Verbindungselement (7) und das Rohr (8) durch Schweißen, insbesondere Laserschweißen, miteinander verbunden sind.

11. Medizinisches Instrument nach einem der Ansprüche 8 bis 10, **dadurch gekennzeichnet, dass** der Außendurchmesser des Verbindungselements (7) nur geringfügig kleiner ist als der Innendurchmesser des Rohres (8).

12. Medizinisches Instrument nach einem der Ansprüche 1 bis 11, **dadurch gekennzeichnet, dass** das Verbindungselement (7) aus Federstahl oder einer superelastischen Verbindung, wie beispielsweise Ni-Ti-, Cu-Al-Ni- oder Cu-Zn-Al-Legierungen, besteht.

13. Medizinisches Instrument nach einem der Ansprüche 1 bis 12, **dadurch gekennzeichnet, dass** das Verbindungselement (7) mehradrig aus mindestens zwei miteinander verdrehten Adern bestehend aufgebaut ist.

14. Medizinisches Instrument nach Anspruch 13, **dadurch gekennzeichnet, dass** die einzelnen Adern des Verbindungselements (7) an einzelnen Stellen durch Verquetschen oder Verschweißen miteinander verbunden sind.

15. Medizinisches Instrument nach einem der Ansprüche 1 bis 14, **dadurch gekennzeichnet, dass** der Ventilkörper (6), aus Hartkunststoff besteht.

16. Medizinisches Instrument nach einem der Ansprüche 1 bis 15, **dadurch gekennzeichnet, dass** am Ventilkörper (6) ein Dichtring (9), insbesondere aus einem Elastomer-Kunststoff, festlegbar ist.

17. Medizinisches Instrument nach Anspruch 16, **dadurch gekennzeichnet, dass** den Instrumentenkanal (4) koaxial umgebend am Gehäuse (1a) eine Dichtfläche zur Auflage des Dichtringes (9) des Ventilkörpers (6) ausgebildet ist.

18. Medizinisches Instrument nach Anspruch 17, **dadurch gekennzeichnet, dass** die Dichtfläche als erhöhte Dichtkante (10), Stufe oder plane Fläche ausgebildet ist.

19. Medizinisches Instrument nach einem der Ansprüche 1 bis 18, **dadurch gekennzeichnet, dass** der Ventilköper (6) zusätzlich über einen manuell betätigbaren Mechanismus in die Offenstellung verlagerbar ist.

20. Medizinisches Instrument nach Anspruch 19, **dadurch gekennzeichnet, dass** der manuell betätigbare Mechanismus als exzentrisch zum Instrumentenkanal angeordneter Stößel (11) ausgebildet ist, der über ein am Gehäuse (1a) gelagertes Betätigungselement in Längsrichtung des Instrumentenkanals (4) verschiebbar ist.

21. Medizinisches Instrument nach Anspruch 20, **dadurch gekennzeichnet, dass** der Stößel (11) parallel zum Instrumentenkanal (4) angeordnet ist.

22. Medizinisches Instrument nach Anspruch 20 oder 21, **dadurch gekennzeichnet, dass** das Betätigungselement als parallel zum Instrumentenkanal (4) verschiebbare Scheibe (12) ausgebildet ist.

23. Medizinisches Instrument nach Anspruch 22, **dadurch gekennzeichnet, dass** die Scheibe (12) über eine Feder (13) in Schließrichtung des Ventilkörpers (6) vorgespannt ist.

24. Medizinisches Instrument nach Anspruch 20 oder 21, **dadurch gekennzeichnet, dass** das Betätigungselement als verschwenkbar am Gehäuse (1a) gelagerter Hebel (15) ausgebildet ist.

25. Medizinisches Instrument nach Anspruch 24, **dadurch gekennzeichnet, dass** der Hebel (15) über eine Feder (16) in Schließrichtung des Ventilkörpers (6) vorgespannt ist.

26. Medizinisches Instrument nach einem der Ansprüche 1 bis 25, **dadurch gekennzeichnet, dass** die Ebene der Ventilöffnung senkrecht zur Längsachse des Instrumentenkanals (4) liegt.

27. Medizinisches Instrument nach einem der Ansprüche 1 bis 26, **dadurch gekennzeichnet, dass** am Gehäuse (1a) ein weiteres Bauteil (2) derart festlegbar ist, dass das weitere Bauteil (2) die Ringnut (1b) bzw. die Ringnutabschnitte (1c) zumindest teilweise verschließend das Verbindungselement (7) in der Ringnut (1b) bzw. den Ringnutabschnitten (1c) hält.

28. Medizinisches Instrument nach Anspruch 27, **dadurch gekennzeichnet, dass** das Gehäuse (1a) ein Trokarkopf (1) und das weitere Bauteil (2) eine am Trokarkopf (1) festlegbare Trokarhülse (2) eines Trokars ist.

## Claims

1. Medical instrument with a hollow instrument channel (4) which is formed in a housing (1a) and can be closed by at least one valve body (6) and can be pivoted into an open position by an instrument which can be inserted into the instrument channel (4), the at least one valve body (6) being designed as a flap which is made of a substantially flexurally rigid material and is mounted on the housing (1a) so as to be pivotable, via a flexurally elastic connection element (7) designed as a wire spring, in such a way that the valve body (6) is pre-tensioned in the closure direction, **characterized in that** the connection element (7) is secured at one end on the housing (1a) and at the other end carries the valve body (6), said connection element (7) being secured in an annular groove (1b) formed in the housing (1a).

2. Medical instrument according to Claim 1, **characterized in that** the annular groove (1b) formed in the housing (1a) in order to secure the connection element (7) is designed as an interrupted annular groove.

3. Medical instrument according to Claim 2, **characterized in that** at least one positioning element (17) interacting with at least one annular groove segment (1c) is arranged on the connection element (7).

4. Medical instrument according to Claim 1, **characterized in that** the annular groove (1b) formed in the housing (1a) in order to secure the connection element (7) is designed as a complete annular groove.

5. Medical instrument according to one of Claims 1 to 4, **characterized in that** the valve body (6) has an annular groove (6a), in particular a complete annular groove, for receiving the connection element (7).

6. Medical instrument according to one of Claims 1 to 5, **characterized in that** the valve body (6) consists of at least two parts which, after being connected to one another, in particular by being wedged, screwed or adhesively bonded to one another, surround a corresponding segment (7c, 7e) of the connection element (7).

7. Medical instrument according to one of Claims 1 to 6, **characterized in that** the connection element (7) is incorporated into the material of the valve body (6), in particular by casting.

8. Medical instrument according to one of Claims 1 to 7, **characterized in that** the part of the connection element (7) that can be secured in the annular groove (1b) in the housing (1a) is surrounded by a tube (8) made of non-elastic material, the connection element (7) and the tube (8) being joined to one another with a force fit.

9. Medical instrument according to Claim 8, **characterized in that** the connection element (7) and the tube (8) are squeezed together.

10. Medical instrument according to Claim 8, **characterized in that** the connection element (7) and the tube (8) are joined to one another by welding, in particular by laser welding.

11. Medical instrument according to one of Claims 8 to 10, **characterized in that** the external diameter of the connection element (7) is only slightly smaller than the internal diameter of the tube (8).

12. Medical instrument according to one of Claims 1 to 11, **characterized in that** the connection element (7) is made of spring steel or of a super-elastic compound such as Ni-Ti, Cu-Al-Ni or Cu-Zn-Al alloys.

13. Medical instrument according to one of Claims 1 to 12, **characterized in that** the connection element (7) is of multi-strand construction, consisting of at least two intertwined strands.

14. Medical instrument according to Claim 13, **characterized in that** the individual strands of the connection element (7) are joined to one another at separate locations by being squeezed or welded together.

15. Medical instrument according to one of Claims 1 to 14, **characterized in that** the valve body (6) is made of hard plastic.

16. Medical instrument according to one of Claims 1 to 15, **characterized in that** a sealing ring (9), in particular made of an elastomeric plastic, can be secured on the valve body (6).

17. Medical instrument according to Claim 16, **characterized in that** a sealing surface for supporting the sealing ring (9) of the valve body (6) is formed on the housing (1a) and coaxially surrounds the instrument channel (4).

18. Medical instrument according to Claim 17, **characterized in that** the sealing surface is designed as a raised sealing edge (10), step, or plane surface.

19. Medical instrument according to one of Claims 1 to 18, **characterized in that** the valve body (6) can additionally be moved to the open position via a manually activatable mechanism.

20. Medical instrument according to Claim 19, **characterized in that** the manually activatable mechanism is designed as a ram (11) which is arranged eccentrically with respect to the instrument channel and which can be moved in the longitudinal direction of the instrument channel (4) via an operating element mounted on the housing (1a).

21. Medical instrument according to Claim 20, **characterized in that** the ram (11) is arranged parallel to the instrument channel (4).

22. Medical instrument according to Claim 20 or 21, **characterized in that** the operating element is designed as a disc (12) which can be moved parallel to the instrument channel (4).

23. Medical instrument according to claim 22, **characterized in that** the disc (12) is pre-tensioned in the closure direction of the valve body (6) via a spring (13).

24. Medical instrument according to Claim 20 or 21, **characterized in that** the operating element is designed as a lever (15) mounted pivotably on the housing (1a).

25. Medical instrument according to Claim 24, **characterized in that** the lever (15) is pre-tensioned in the closure direction of the valve body (6) via a spring (16).

26. Medical instrument according to one of Claims 1 to 25, **characterized in that** the plane of the valve aperture lies perpendicular to the longitudinal axis of the instrument channel (4).

27. Medical instrument according to one of Claims 1 to 26, **characterized in that** a further component (2) can be secured on the housing (1a) in such a way that said further component (2), at least partially closing the annular groove (1b) or annular groove segments (1c), holds the connection element (7) in the annular groove (1b) or annular groove segments (1c).

28. Medical instrument according to Claim 27, **characterized in that** the housing (1a) is a trocar head (1), and the further component (2) is a trocar sleeve (2) of a trocar and can be secured on the trocar head (1).

## Revendications

1. Instrument médical présentant un canal creux à instrument (4) ménagé dans un boîtier (1a) et susceptible d'être refermé par au moins un corps de valve (6) et mobile en pivotement jusque dans une position d'ouverture par un instrument enfichable dans le canal à instrument (4), ledit au moins un corps de valve (6) étant réalisé sous forme de clapet constitué en matériau sensiblement rigide en flexion et monté avec faculté de pivotement sur le boîtier (1a) via un élément de liaison (7) élastique en flexion réalisé sous forme de ressort en fil, de telle sorte que le corps de valve (6) est précontraint en direction de fermeture,
**caractérisé en ce que**
l'élément de liaison (7) est d'une part immobilisé sur le boîtier (1a) et porte d'autre part le corps de valve (6), l'élément de liaison (7) étant immobilisé dans une gorge annulaire (1b) ménagée dans le boîtier (1a).

2. Instrument médical selon la revendication 1, **caractérisé en ce que** la gorge annulaire (1b) ménagée dans le boîtier (1a) et destinée à immobiliser l'élément de liaison (7) est réalisée sous forme de gorge annulaire interrompue.

3. Instrument médical selon la revendication 2, **caractérisé en ce qu'**au moins un élément de positionnement (17) coopérant avec au moins un tronçon (1c) de la gorge annulaire est agencé sur l'élément de liaison (7).

4. Instrument médical selon la revendication 1, **caractérisé en ce que** la gorge annulaire (1b) ménagée dans le boîtier (1a) et destinée à immobiliser l'élément de liaison (7) est réalisée sous forme de gorge annulaire périphérique.

5. Instrument médical selon l'une des revendications 1 à 4, **caractérisé en ce que** le corps de valve (6) présente une gorge annulaire (6a), en particulier une gorge annulaire périphérique pour recevoir l'élément de liaison (7).

6. Instrument médical selon l'une des revendications 1 à 5, **caractérisé en ce que** le corps de valve (6) est constitué par au moins deux parties qui, une fois reliées l'une à l'autre en particulier par coincement, vissage ou collage, enferment un tronçon correspondant (7c, 7e) de l'élément de liaison (7).

7. Instrument médical selon l'une des revendications 1 à 6, **caractérisé en ce que** l'élément de liaison (7) est intégré, en particulier surmoulé dans le matériau du corps de valve (6).

8. Instrument médical selon l'une des revendications 1 à 7, **caractérisé en ce que** la partie de l'élément de liaison (7) susceptible d'être immobilisée dans la gorge annulaire (1b) dans le boîtier (1a) est entourée par un tube (8) en matériau inélastique, l'élément de liaison (7) et le tube (8) étant reliés en coopération de forces l'un à l'autre.

9. Instrument médical selon la revendication 8, **caractérisé en ce que** l'élément de liaison (7) et le tube (8) sont reliés l'un à l'autre par écrasement.

10. Instrument médical selon la revendication 8, **caractérisé en ce que** l'élément de liaison (7) et le tube (8) sont reliés l'un à l'autre par soudage, en particulier par soudage au laser.

11. Instrument médical selon l'une des revendications 8 à 10, **caractérisé en ce que** le diamètre extérieur de l'élément de liaison (7) n'est que peu inférieur au diamètre intérieur du tube (8).

12. Instrument médical selon l'une des revendications 1 à 11, **caractérisé en ce que** l'élément de liaison (7) est constitué en acier ressort ou en une composition super-élastique, comme par exemple des alliages Ni-Ti, Cu-Al-Ni ou Cu-Zn-Al.

13. Instrument médical selon l'une des revendications 1 à 12, **caractérisé en ce que** l'élément de liaison (7) est constitué en plusieurs brins, en particulier à partir d'au moins deux brins mutuellement torsadés.

14. Instrument médical selon la revendication 13, **caractérisé en ce que** les brins individuels de l'élément de liaison (7) sont reliés l'un à l'autre par écrasement ou soudage à des emplacements individuels.

15. Instrument médical selon l'une des revendications 1 à 14, **caractérisé en ce que** le corps de valve (6) est constitué en matière plastique dure.

16. Instrument médical selon l'une des revendications 1 à 15, **caractérisé en ce qu'**un anneau d'étanchéité (9), en particulier en matière plastique élastomère est susceptible d'être immobilisé sur le corps de valve (6).

17. Instrument médical selon la revendication 16, **caractérisé en ce qu'**une surface d'étanchéité pour supporter l'anneau d'étanchéité (9) du corps de valve (6) est réalisée sur le boîtier (1a) en entourant coaxialement le canal à instrument (4).

18. Instrument médical selon la revendication 17, **caractérisé en ce que** la surface d'étanchéité est réalisée sous la forme d'une arête d'étanchéité surélevée (10), d'un gradin ou d'une surface plane.

19. Instrument médical selon l'une des revendications 1 à 18, **caractérisé en ce que** le corps de valve (6) est déplaçable jusque dans la position d'ouverture en supplément par un mécanisme à actionnement manuel.

20. Instrument médical selon la revendication 19, **caractérisé en ce que** le mécanisme à actionnement manuel est réalisé sous la forme d'un poussoir (11) agencé excentriquement par rapport au canal à instrument et est mobile en translation en direction longitudinale du canal à instrument (4) par un élément d'actionnement monté sur le boîtier (1a).

21. Instrument médical selon la revendication 20, **caractérisé en ce que** le poussoir (11) est agencé parallèlement au canal à instrument (4).

22. Instrument médical selon l'une ou l'autre des revendications 20 et 21, **caractérisé en ce que** l'élément d'actionnement est réalisé sous la forme d'un disque (12) mobile en translation parallèlement au canal à instrument (4).

23. Instrument médical selon la revendication 22, **caractérisé en ce que** le disque (12) est précontraint en direction de fermeture du corps de valve (6) par un ressort (13).

24. Instrument médical selon l'une ou l'autre des revendications 20 et 21, **caractérisé en ce que** l'élément d'actionnement est réalisé sous la forme d'un levier (15) monté avec faculté de pivotement sur le boîtier (1a).

25. Instrument médical selon la revendication 24, **caractérisé en ce que** le levier (15) est précontraint en direction de fermeture du corps de valve (6) par un ressort (16).

26. Instrument médical selon l'une des revendications 1 à 25, **caractérisé en ce que** le plan de l'ouverture de valve est perpendiculaire à l'axe longitudinal du canal à instrument (4).

27. Instrument médical selon l'une des revendications 1 à 26, **caractérisé en ce qu'**un autre composant (2) est susceptible d'être immobilisé sur le boîtier (1a) de telle sorte que l'autre composant (2) maintient l'élément de liaison (7) dans la gorge annulaire (1b) ou dans les tronçons (1c) de la gorge annulaire en refermant au moins partiellement la gorge annulaire (1b) ou les tronçons (1c) de la gorge annulaire.

28. Instrument médical selon la revendication 27, **caractérisé en ce que** le boîtier (1a) est une tête de trocart (1) et l'autre composant (2) est une douille (2) d'un trocart, laquelle est susceptible d'être immobilisée sur la tête de trocart (1).
